# EUROPÄISCHE PATENTSCHRIFT

(11) **EP 0 991 444 B1**
(45) Veröffentlichungstag und Bekanntmachung des Hinweises auf die Patenterteilung: **09.02.2005**
(21) Anmeldenummer: 98937486.3
(22) Anmeldetag: 16.06.1998
(51) Int. Cl.: A61M 25/06

(54) **KANÜLENANORDNUNG**
CANNULAR ARRANGEMENT
SYSTEME DE CANULE

(30) Priorität: 18.06.1997 DE 19725680
(43) Veröffentlichungstag der Anmeldung: 12.04.2000
(73) Patentinhaber: Haindl, Hans, Dr., 30974 Wennigsen (DE)
(72) Erfinder: Haindl, Hans, Dr., 30974 Wennigsen (DE)
(74) Vertreter: Leine, Sigurd, Dipl.-Ing.
(86) Internationale Anmeldenummer: PCT/EP1998/003620
(87) Internationale Veröffentlichungsnummer: WO 1998/057693

(56) Entgegenhaltungen:
- EP-A- 0 567 321
- EP-A- 0 599 564
- EP-A- 0 646 386

## Beschreibung

Die Erfindung betrifft eine Kanülenanordnung der im Oberbegriff des Anspruchs 1 genannten Art zum Einführen eines Katheters in biologisches Gewebe oder dergleichen.

Durch EP 0 567 321 A2 ist eine Kanülenanordnung zum Einführen eines Katheters in biologisches Gewebe oder dergleichen bekannt, die eine Kanüle aus Stahl mit einem vorderen, spitzen Ende und mit einem hinteren Ende aufweist, an dem die Kanüle von einem Ansatz aus Kunststoff gehalten ist. Ferner weist die bekannte Kanülenanordnung ein Gehäuse aus Kunststoff auf, das an seinem in Einstichrichtung vorderen Ende eine vordere Öffnung und an seinem in Einstichrichtung hinteren Ende eine hintere Öffnung aufweist. An dem Gehäuse ist der Ansatz zwischen einer vorgeschobenen Lage, in der das vordere Ende der Kanüle in Axialrichtung über das Gehäuse übersteht, und einer zurückgezogenen Lage, in der die Kanüle in dem Gehäuse aufgenommen ist, in Axialrichtung der Kanüle verschieblich gehalten.

Die bekannte Kanülenanordnung wird mit einem dünnen Rohr verwendet, das einen Ansatz aufweist, mit dem das Rohr mit dem Gehäuseteil der Kanülenanordnung lösbar verbunden ist und das die Kanüle umgibt.

Zum Einführen eines Katheters in biologisches Gewebe, beispielsweise den Körper eines Patienten, mittels der Kanülenanordnung befindet sich die Kanüle in ihrer vorgeschobenen Lage, in der das spitze Ende der Kanüle in Einstichrichtung über das Gehäuse und über das vordere Ende des Rohres übersteht, so daß die Kanüle in die Haut des Patienten eingestochen und durch die Einstichöffnung das dünne Rohr in den Körper des Patienten eingeführt werden kann.

Nach Einführen des Rohres verschiebt der Benutzer den Ansatz mit der Kanüle in die zurückgezogene Lage, in der die Kanüle in dem Gehäuse aufgenommen ist, und trennt das Gehäuse von dem Rohr. Das Verschieben der Kanüle in ihre zurückgezogene Lage dient ausschließlich dazu, eine Berührung des spitzen Endes der Kanüle und damit möglicherweise verbundene Verletzungen sowie Infektionen zu vermeiden.

Durch das im Körper des Patienten verbleibende Rohr kann dann unmittelbar infundiert oder ein Katheter aus flexiblem Material in den Körper des Patienten eingeführt werden, durch den Flüssigkeiten in den Körper des Patienten infundiert oder aus dem Körper des Patienten entnommen werden können.

Ein Nachteil der bekannten Kanülenanordnung besteht darin, daß nach Einführen des Rohres die Kanülenanordnung von dem Rohr gelöst werden muß, bevor ein zuführender Schlauch aus flexiblem Material an das Rohr angeschlossen werden kann. Hierfür sind weitere Arbeitsschritte erforderlich, was die Handhabung weiter kompliziert.

Durch PCT/US95/05671 ist eine Kanülenanordnung der betreffenden Art bekannt, bei der der Katheter fest mit dem vorderen Ende des Gehäuses verbunden ist und dort endet. Die Kanüle bleibt im zurückgezogenen Zustand des Schiebers mit ihrem vorderen Ende in dem Katheter und stellt so eine Fluidverbindung zwischen dem Katheter und dem hohlen Inneren des Schiebers her, der an seinem hinteren Ende einen Anschluß für einen Schlauch aufweist.

Diese bekannte Kanülenanordnung hat den Nachteil, daß sie nur mit Kathetern mit offener Spitze möglich ist, nicht hingegen bei Kathetern mit geschlossener Spitze und mehrlumigen Kathetern. Außerdem hat das System einen großen Totraum und Dichtigkeitsprobleme.

Durch WO 95/20991 ist eine Anordnung zur Applikation von Mikrodialysesonden bekannt, bei der die Mikrodialysesonde zunächst in einer Kanüle mit Längsschlitz angeordnet und diese Kombination in Gewebe eingestochen wird. Nachdem sich der Mikrodialysekatheter in dem Gewebe befindet, wird die Kanüle zurückgezogen und über den Schlitz von dem Fluidkanal des Mikrodialysekatheters getrennt.

Durch WO 96/25088 ist eine Vorrichtung zur Applikation von Sensoren bekannt, bei der ebenfalls eine geschlitzte Nadel erforderlich ist, die nach Applikation des Sensors von dem Sensor getrennt werden kann.

Beide bekannten Anordnungen sind wegen der Verwendung einer geschlitzten Kanüle aufwendig in der Herstellung und unpraktisch im Gebrauch.

Der Erfindung liegt die Aufgabe zugrunde, eine Kanülenanordnung der betreffenden Art zu schaffen, die für Katheter mit geschlossener Spitze und mehrlumige Katheter, beispielsweise aus DE 197 14 572 A1 oder DE 33 42 170 bekannte Mikrodialysekatheter, geeignet ist und bei der keine Undichtigkeiten auftreten.

Der Grundgedanke der erfindungsgemäßen Lehre besteht darin, den Katheter durch den Schieber und das Gehäuse bis zu dessen hinteren Ende zu führen und dort entgegen der Einstechrichtung zu befestigen, wo der Katheter unmittelbar an Schläuche angeschlossen werden kann. Besonders vorteilhafterweise setzt sich der Katheter über das hintere Ende des Gehäuses fort. Undichtigkeiten können an keiner Stelle auftreten.

Da der Katheter nur entgegen der Einstechrichtung unbeweglich mit dem Gehäuse und/oder dem Schieber verbunden ist, kann er nicht versehentlich nach hinten aus dem Gehäuse herausgezogen werden, gleichzeitig ist es jedoch möglich, ihn in Einstechrichtung nach dem Einstechen beliebig weit über die Spitze der Kanüle hinaus vorzuschieben. Ist der Katheter in Einstechrichtung fest mit dem Schieber verbunden, so ist es möglich, die Kanüle vor der Anwendung in der zurückgezogenen Lage zu belassen, wo ihre Spitze geschützt ist. Vor Anwendung wird der Schieber vorgeschoben, wobei der Katheter mitgenommen wird.

Der Katheter kann aber auch völlig fest mit dem hinteren Ende des Gehäuses verbunden sein, wobei er sich dann bis zur Spitze der Kanüle erstreckt, wenn sich diese in ihrer vorgeschobenen Lage befindet.

Zwar ist es grundsätzlich möglich, den Schieber während des Einstechens der Kanüle von Hand in Bezug auf das Gehäuse zu halten, jedoch sieht eine zweckmäßige Ausführungsform der Erfindung Verriegelungsmittel zum Verriegeln des Schiebers an dem Gehäuse wenigstens in der vorgeschobenen Lage des Schiebers vor. Das Einstechen kann dann allein durch Halten des Gehäuses erfolgen. Danach ist dann eine Entriegelung und ein Zurückziehen des Schiebers möglich. Hierzu sind gemäß einer Ausführungsform der Erfindung Mittel zum Zurückziehen der Kanüle von Hand vorgesehen. Hierzu ist es vorteilhaft, daß das Gehäuse einen sich in Verschieberichtung der Kanüle erstreckenden Schlitz aufweist, durch den sich ein radialer Vorsprung des Schiebers erstreckt. Mittels dieses von außen zugänglichen Vorsprunges kann dann das Zurückschieben des Schiebers erfolgen.

Gemäß einer Ausführungsform der Erfindung weist der Schieber einen um eine Achse quer zu seiner Schieberichtung kippbaren, zweiarmigen Hebel auf, dessen vorderer Hebelarm in der vorderen Endlage des Schiebers eine Kante am vorderen Ende des Gehäuses hintergreift und der durch Druck auf den hinteren Hebelarm ausrastbar ist. Der doppelarmige Hebel stellt also ein Betätigungsglied sowohl zum Ent- und Verriegeln als auch zum Verschieben des Schiebers dar.

Gemäß einer besonders zweckmäßigen Ausführungsform der Erfindung ist eine Feder vorgesehen, die den Schieber in Richtung auf seine hintere Endlage drückt, wobei der Schieber in seiner vorderen Endlage durch eine von außerhalb des Gehäuses lösbare Rastung gehalten ist. Bei dieser Ausführungsform besorgt die Feder das Zurückschieben des Schiebers und der Kanüle, wodurch die Handhabung erleichtert ist.

Bei Verwendung der Feder weist die Rastung zweckmäßigerweise einen Haken auf, der mit dem Schieber verbunden ist, in der vorderen Endlage des Schiebers eine Kante am vorderen Ende des Gehäuses hintergreift und in Entriegelungsrichtung nachgiebig ist. Diese grundsätzliche Ausführungsform der Rastung ermöglicht eine Weiterbildung, gemäß der der Haken einen über das vordere Ende des Gehäuses vorstehenden Vorsprung aufweist, derart, daß der Vorsprung bei Einstechen der Kanüle gegen die Gewebeoberfläche stößt und den Haken entriegelt. Bei dieser Ausführungsform erfolgt das Zurückziehen der Kanüle praktisch selbsttätig, so daß einer Entriegelung und einem Zurückschieben keine besondere Aufmerksamkeit geschenkt zu werden braucht.

Die Entriegelungsmittel können in vielfältiger Weise ausgebildet sein, und der minimale Abstand zwischen einem Hindernis und dem Gehäuseteil, bei dessen Erreichen die Entriegelungsmittel entriegeln, ist in weiten Grenzen wählbar. Gemäß einer zweckmäßigen Weiterbildung der Ausführungsform mit den Verriegelungsmitteln weisen die Verriegelungsmittel eine mit dem Ansatz verbundene Verriegelungszunge auf, die in Verriegelungslage mit einem radialen Vorsprung federnd in eine radiale Ausnehmung im Inneren des Gehäuseteils eingreift, wobei die Entriegelungsmittel ein relativ zu dem Gehäuseteil axial verschiebliches Entriegelungselement aufweisen, dessen vorderes Ende axial über das vordere Ende des Gehäuseteiles vorsteht und dessen hinteres Ende eine Entriegelungszunge aufweist, die sich in das Innere des Gehäuseteiles erstreckt und bei axialer Verschiebung des Entriegelungselementes in Richtung auf das Gehäuseteil die Verriegelungszunge untergreift und aus der Ausnehmung herausbewegt. Diese Ausführungsform ist einfach und kostengünstig herstellbar und zuverlässig in der Funktion.

Gemäß einer Weiterbildung der vorgenannten Ausführungsform liegen die Entriegelungszunge und die Verriegelungszunge in Ruhelage mit Abschrägungen aneinander an, derart, daß die Entriegelungszunge bei axialer Verschiebung des Entriegelungselements in Richtung auf das Gehäuseteil die Verriegelungszunge anhebt. Diese Ausführungsform ist einfach im Aufbau und kostengünstig herstellbar.

Eine andere Weiterbildung der Ausführungsform mit Entriegelungszunge und Verriegelungszunge sieht vor, daß jeweils wenigstens zwei Entriegelungszungen und zugeordnete Verriegelungszungen an in Bezug zu der Kanüle diametral gegenüberliegenden Stellen des Entriegelungselementes bzw. des Ansatzes vorgesehen sind. Bei dieser Ausführungsform ist ein Verkanten des Entriegelungselementes verhindert, so daß die Zuverlässigkeit der Entriegelungsmittel verbessert ist.

Gemäß einer anderen Weiterbildung der Ausführungsformen mit dem Entriegelungselement ist dessen vorderes Ende im wesentlichen ringförmig ausgebildet. Auf diese Weise ist unabhängig von der Drehlage des Gehäuseteiles um die Kanülenachse beim Auftreffen des Entriegelungselementes auf ein Hindernis stets eine sichere Entriegelung erzielt.

Eine andere Ausführungsform der erfindungsgemäßen Lehre sieht vor, daß die Feder eine zwischen dem Gehäuse und dem Ansatz angeordnete Wendelfeder, insbesondere eine zu der Kanüle koaxiale Druckfeder, ist. Diese Ausführungsform ist besonders einfach im Aufbau und kostengünstig herstellbar. Durch entsprechende Dimensionierung der Wendelfeder ist die nach Entriegelung auf den Schieber in Richtung auf die zurückgezogene Lage wirkende Kraft wählbar.

Eine andere Ausführungsform sieht Haltemittel zum Halten des Gehäuses auf einer Körperoberfläche, beispielsweise der Haut eines Menschen, vor. Bei dieser Ausführungsform ist die Kanülenanordnung nach dem Einstechen der Kanüle und Zurückziehen der Kanüle in ihre zurückgezogene Lage beispielsweise auf der Haut eines Menschen gehalten, so daß eine Belastung des Katheters durch das Gewicht der auf dem Katheter verbleibenden Kanülenanordnung vermieden ist.

Gemäß einer Weiterbildung der Ausführungsform mit den Haltemitteln weisen diese auf der Außenseite des Gehäuses angeordnete Adhäsivmittel, insbesondere einen Klebestreifen, auf. Bei dieser Ausführungsform haftet das Gehäuse auf der Haut und kann später, wenn der Katheter aus dem Körper des Patienten entfernt wird, wieder abgenommen werden.

Zweckmäßigerweise weist das Gehäuseteil eine Abflachung zur Anlage an der Körperoberfläche, beispielsweise der Haut eines Menschen, auf. Hierbei liegt das Gehäuseteil flächig auf der Haut auf, so daß Druckstellen oder dergleichen auf der Haut vermieden sind.

Schließlich sieht eine weitere Ausführungsform der erfindungsgemäßen Lehre vor, daß die vordere Öffnung des Gehäuseteiles eine lichte Weite aufweist, die im wesentlichen dem Außendurchmesser der Kanüle entspricht. Bei dieser Ausführungsform ist einerseits die Kanüle in ihrer vorgeschobenen Lage seitlich geführt, so daß ein seitliches Ausweichen der Kanüle beim Einstechen verhindert ist. Zum anderen ist bei zurückgezogener Kanüle der Katheter in der vorderen Öffnung in Kanülenrichtung geführt. Hierdurch sind Beschädigungen vermieden, die entstehen könnten, wenn ein flexibler Katheter unmittelbar hinter der Kanülenspitze schräg zu dieser abknickt und dadurch mit seiner Außenfläche an der Kanülenspitze anliegt.

Anhand der Zeichnungen soll die Erfindung an Ausführungsbeispielen nachfolgend näher erläutert werden.
- Fig. 1: zeigt in teilweise geschnittener Seitenansicht ein erstes Ausführungsbeispiel der erfindungsgemäßen Kanülenanordnung, wobei sich die Kanüle in ihrer vorgeschobenen Lage befindet,
- Fig. 2: zeigt eine Draufsicht von Fig. 1,
- Fig. 3: entspricht Fig. 1, wobei sich die Kanüle jedoch in ihrer zurückgezogenen Stellung befindet,
- Fig. 4: zeigt eine Draufsicht von Fig. 3 auf die Kanülenanordnung gemäß Fig. 3,
- Fig. 5: zeigt einen vergrößerten Schnitt V-V durch Fig. 1,
- Fig. 6-9: verdeutlichen die Verwendung der Kanülenanordnung gemäß Fig. 1-5,
- Fig. 10: zeigt eine Seitensicht eines zweiten Ausführungsbeispiels der erfindungsgemäßen Kanülenanordnung, wobei sich die Kanüle in ihrer vorgeschobenen Lage befindet,
- Fig. 11: zeigt die Kanülenanordnung gemäß Fig. 10, wobei sich die Kanüle in ihrer zurückgezogenen Lage befindet,
- Fig. 12: zeigt eine Schnittansicht A-A in Fig. 11,
- Fig. 13: zeigt eine Schnittansicht B-B in Fig. 11 und
- Fig. 14: zeigt einen Schnitt durch Fig. 10 im Bereich eines Kreises K in Fig. 10,
- Fig. 15: zeigt im Schnitt ein drittes Ausführungsbeispiel der Erfindung mit vorgeschobener Kanüle und
- Fig. 16: entspricht Fig. 15, jedoch mit zurückgezogener Kanüle.

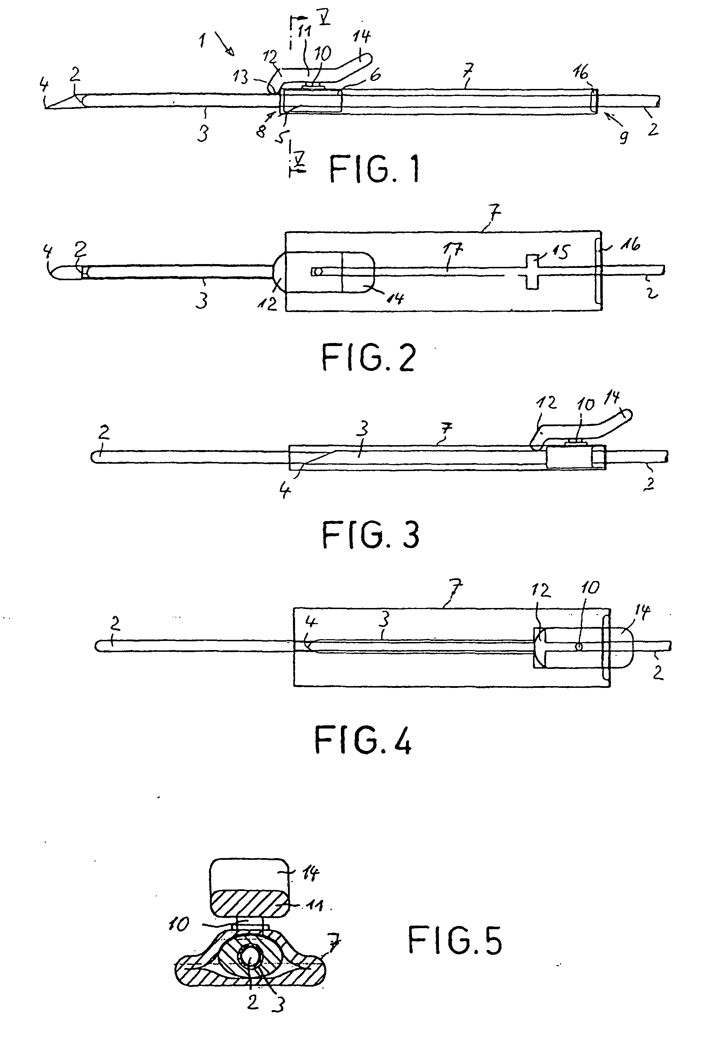

In Fig. 1 ist eine Kanülenanordnung 1 zum Einführen eines aus einem flexiblen Material bestehenden, als Mikrodialysekatheter ausgebildeten Katheters 2 in biologisches Gewebe oder dergleichen dargestellt, die eine Kanüle 3 mit einem vorderen spitzen Ende 4 und einem hinteren Ende 5 aufweist, an dem die Kanüle 3 von einem hülsenförmigen Schieber 6 aus Kunststoff gehalten ist. Die Kanülenanordnung 1 weist ferner ein rohrförmiges Gehäuse 7 aus Kunststoff auf, das im Bereich seines in Einstichrichtung vorderen Endes eine vordere Öffnung 8 und im Bereich seines in Einstichrichtung hinteren Endes eine hintere Öffnung 9 aufweist, durch die der Katheter 2 geführt und in der er fest mit dem Gehäuse 7 verbunden ist. In dem Gehäuse 7 ist der Schieber 6 zwischen einer vorgeschobenen Lage, die in Fig. 1 dargestellt ist und in der das vordere Ende 4 der Kanüle 3 in Axialrichtung über das Gehäuse 7 übersteht, und einer zurückgezogenen Lage, in der die Kanüle 3 in dem Gehäuse 7 aufgenommen ist, in Axialrichtung der Kanüle 3 verschieblich gehalten.

Das Gehäuse 7 weist an seiner Oberseite einen in Fig. 1 nicht erkennbaren, in Fig. 2 dargestellten, sich in Verschieberichtung des Schiebers 6 erstreckenden Schlitz 17 auf, durch den sich ein radialer Vorsprung 10 des Schiebers 6 erstreckt, an dessen dem Schieber 6 abgewandten Ende ein zweiarmiger Hebel 11 angeordnet ist. Der Hebel 11 weist einen vorderen Hebelarm 12, an dem eine Rastnase 13 angeordnet ist, und einen hinteren Hebelarm 14 auf, der ein von Hand betätigbares Betätigungselement bildet. In der vorgeschobenen Lage des Schiebers 6 hintergreift die Rastnase 13 das in Einstichrichtung vordere Ende des Gehäuses 7 federnd.

Der Katheter 2 ist mit einem Stopfen 16 in der hinteren Öffnung 9 des Gehäuses 7 gehalten und erstreckt sich durch das Innere des Gehäuses 7 und den Querschlitz 15 in das Innere der Kanüle 3 bis kurz vor deren vorderes Ende 4.

Fig. 2 zeigt eine Ansicht von oben auf die Kanülenanordnung 1 gemäß Fig. 1. In dieser Darstellung ist ein Querschlitz 15 erkennbar, in den die Rastnase 13 in der zurückgezogenen Stellung des Schiebers 6 einrastbar ist.

Fig. 3 zeigt die Kanülenannordnung gemäß den Figuren 1 und 2, bei der jedoch der Schieber 6 zurückgezogen und die Rastnase 13 in den Querschlitz 15 eingerastet ist. In dieser Stellung befindet sich das vordere spitze Ende 4 der Kanüle 3 innerhalb des Gehäuses 7, so daß es keine Verletzungen hervorrufen kann. Der Katheter 3 ragt durch die vordere Öffnung 8 in dem Gehäuse 7 und ist dadurch in der Weise geführt, daß keine Relativbewegungen zwischen dem Katheter 3 und dem scharfen vorderen Ende 4 der Kanüle 3 möglich sind und so der Katheter 3 gegen Beschädigungen geschützt ist.

Fig. 4 zeigt eine Ansicht von oben auf Fig. 3.

Fig. 5 zeigt einen Schnitt V-V durch Fig. 1 in vergrößerter Darstellung. Aus ihr ist ersichtlich, daß das Gehäuse 7 an seiner Oberseite den Schlitz 17 aufweist, in der der Schieber 6 mit seinem radialen Vorsprung 10 geführt ist. Bei dem Katheter 2 handelt es sich um einen Katheter mit einer Kanaltrennung, also um einen zweilumigen Katheter. Die erfindungsgemäße Kanülenanordnung 1 ist jedoch auch mit anderen Kathetern verwendbar.

Das Gehäuse 7 weist an seiner Unterseite eine Abflachung 18 zur Anlage an einer Oberfläche, insbesondere der Haut eines Menschen, auf. Die vordere Öffnung 8 des Gehäuses 7 ist oval mit einer lichten Weite ausgebildet, die größer ist als der Außendurchmesser der Kanüle 3. Die vordere Öffnung 8 kann jedoch auch eine lichte Weite aufweisen, die im wesentlichen dem Außendurchmesser der Kanüle 3 entspricht, so daß die Kanüle 3 und, in ihrer zurückgezogenen Lage, der Katheter 2 eng in der vorderen Öffnung 8 geführt ist. Auf diese Weise ist verhindert, daß in der zurückgezogenen Lage der Kanüle 3 der Katheter 2 unmittelbar hinter dem vorderen Ende 4 der Kanüle 3 abknickt und durch dieses Ende der Kanüle 3 beschädigt wird.

Aus der Zeichnung ist nicht ersichtlich und deshalb wird hier erläutert, daß auf der Außenseite des Gehäuses 7 an der Abflachung 18 ein Klebestreifen angeordnet ist, der ein Haltemittel zum Halten des Gehäuses 7 auf einer Oberfläche, insbesondere der Haut eines Menschen, bildet.

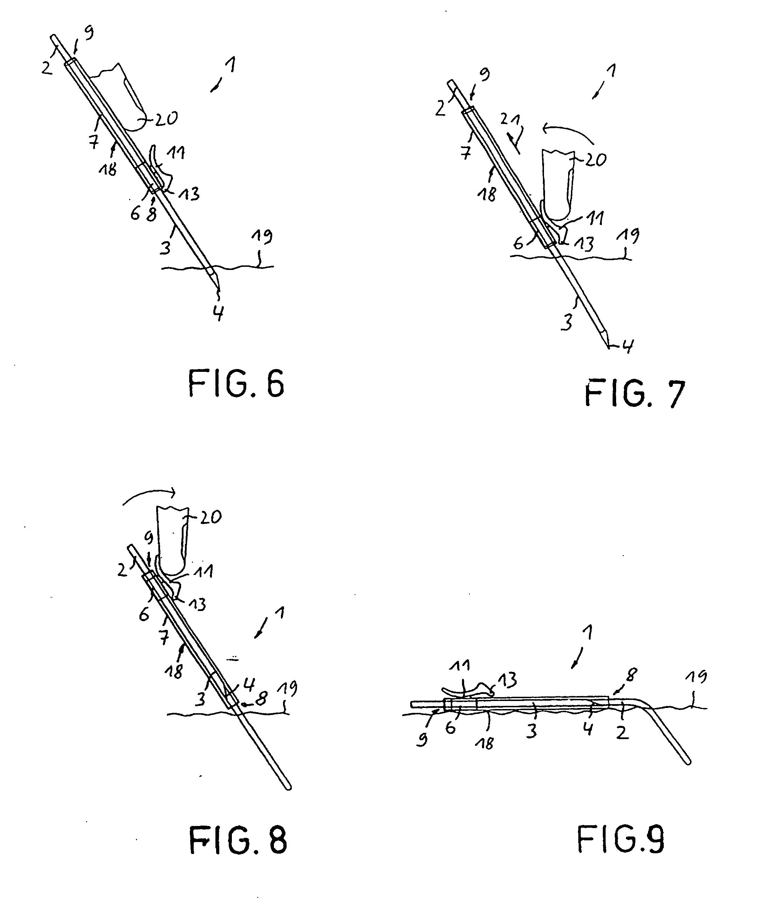

Die Funktionsweise der erfindungsgemäßen Kanülenanordnung 1 soll nachfolgend anhand der Fig. 6 bis 9 erläutert werden, die verschiedene Phasen beim Einführen des Katheters 2 in das Gewebe eines Menschen mittels der Kanülenanordnung 1 darstellen.

Beim Einstechen der Kanüle 1 in die in Fig. 6 durch eine Linie 19 symbolisierte Haut des Patienten befindet sich die Kanüle 3 in ihrer vorgeschobenen Lage, in der ihr vorderes Ende 4 in Axialrichtung über das Gehäuse 7 und das vordere Ende des Katheters 2 übersteht und in der der Schieber 6 mittels der Rastnase 13 an dem Gehäuse 7 verriegelt ist. Zum Einstechen der Kanülenanordnung 1 ergreift ein Benutzer das Gehäuse 7, wobei ein Finger 20 auf dem Gehäuse 7 aufliegt, wie dies in Fig. 6 gezeigt ist, und sticht das vordere Ende 4 der Kanüle 3 in die Haut 19 ein.

Anschließend führt der Benutzer die Kanüle 3 bis zu einer gewünschten Tiefe in das Gewebe ein, wie dies in Fig. 7 dargestellt ist. Dann betätigt der Benutzer mit dem Finger 20 den hinteren Hebelarm 14 des Hebels 11, so daß die Rastnase 13 von dem vorderen Ende des Gehäuseteiles 7 ausrastet und der Schieber 6 mit der Kanüle 3 in Richtung eines Pfeiles 21 in Richtung auf seine zurückgezogene Lage bewegt werden kann.

In Fig. 8 befindet sich der Schieber 6 in seiner zurückgezogenen Lage, in der die Kanüle 3 vollständig in dem Gehäuse 7 aufgenommen ist, so daß eine Verletzung des Patienten oder eine Beschädigung des sich im Körper des Patienten befindlichen Teiles des Katheters 2 durch das vordere Ende 4 der Kanüle 3 zuverlässig vermieden ist. In der zurückgezogenen Lage des Schiebers 6 läßt der Benutzer den Hebel 11 los, so daß dieser mit seiner Rastnase 13 in den Querschlitz 15 des Schlitzes 17 einrastet und somit den Schieber 6 in der zurückgezogenen Lage an dem Gehäuse 7 verriegelt.

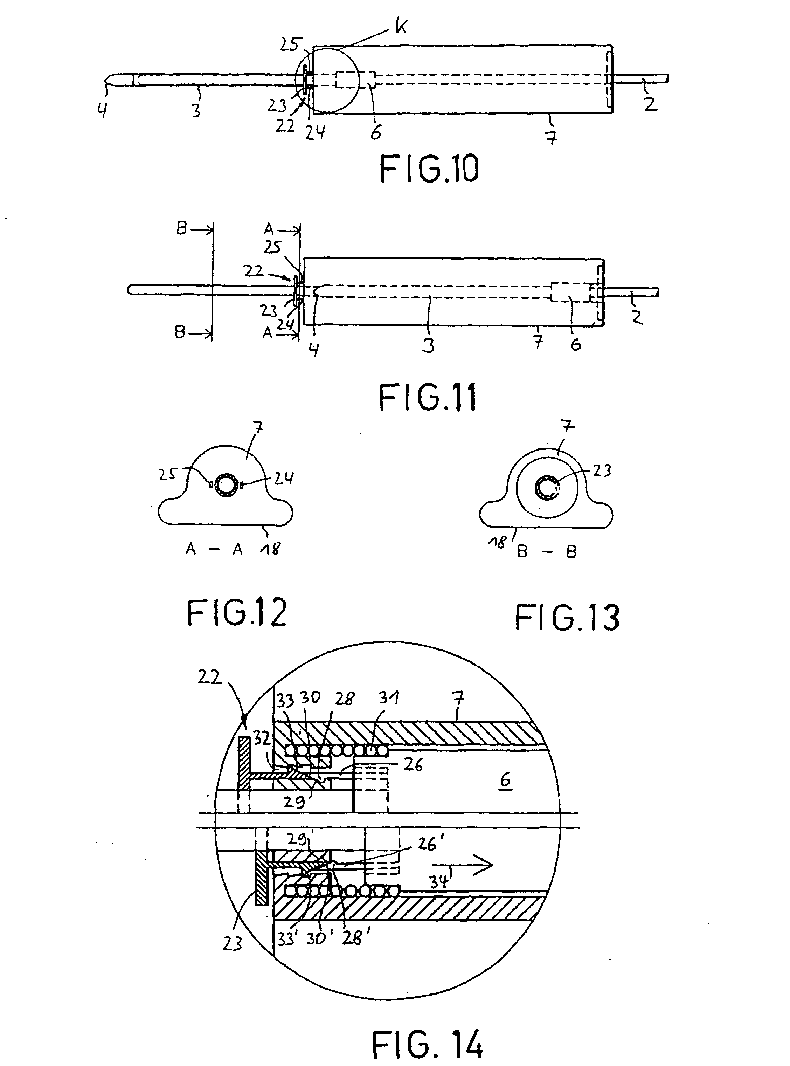

Dann kann eine Schutzfolie des auf der Abflachung 18 angeordneten Klebestreifens abgezogen und das Gehäuse 7 mit seiner Abflachung 18 auf die Haut 19 flach aufgelegt werden, wo der Klebestreifen an der Haut 19 haftet, wie dies in Fig. 9 dargestellt ist.

In der in Fig. 9 dargestellten Lage kann nun mittels des Katheters 2 Flüssigkeit in das Gewebe infundiert oder aus dem Gewebe entnommen werden.

Da die Kanülenanordnung 1 nach dem Einführen des Katheters 2 in das Gewebe auf dem Katheter 2 verbleibt und nicht von diesem gelöst werden muß, ist die Handhabung der erfindungsgemäßen Kanülenanordnung 1 wesentlich vereinfacht und zeitsparender gestaltet.

In Fig. 10 ist ein zweites Ausführungsbeispiel der erfindungsgemäßen Kanülenanordnung dargestellt. Gleiche oder sich entsprechende Teile sind mit gleichen Bezugszeichen versehen.

Das in Fig. 10 dargestellte zweite Ausführungsbeispiel der Kanülenanordnung 1 unterscheidet sich von dem ersten Ausführungsbeispiel gemäß den Fig. 1 bis 9 dadurch, daß die Verriegelungsmittel zur Verriegelung des Schiebers 6 an dem Gehäuse 7 anders ausgebildet sind, wie dies unten bei Fig. 14 näher erläutert wird, und daß Entriegelungsmittel vorgesehen sind, die die Verriegelungsmittel entriegeln, wenn sich die Kanüle 3 in der vorgeschobenen Lage befindet und das in Einstichrichtung vordere Ende des Gehäuses 7 in den Bereich eines Hindernisses, beispielsweise der Haut eines Patienten, gelangt.

In Fig. 10 befindet sich der Schieber 6 mit der Kanüle 3 in seiner vorgeschobenen Lage. Die Entriegelungsmittel weisen ein axial verschieblich an dem Gehäuse 7 gehaltenes Entriegelungselement 22 auf, dessen vorderes Ende 23 ringförmig ausgebildet und zu der Kanüle 3 koaxial ist, und dessen hinteres Ende sich mit Entriegelungszungen 24, 25, die weiter unten zu Fig. 13 näher erläutert werden, in das Innere des Gehäuses 7 erstreckt.

Fig. 11 zeigt die Kanülenanordnung 1 gemäß Fig. 9, wobei sich der Schieber 6 in der zurückgezogenen Lage befindet, in der die Kanüle 3 in dem Gehäuse 7 aufgenommen ist.

Fig. 12 zeigt einen Schnitt A-A in Fig. 11, wobei die Entriegelungszungen 24, 25 erkennbar sind.

Fig. 13 zeigt einen Schnitt B-B in Fig. 11, wobei das ringförmige Ende 23 des Entriegelungselementes 22 erkennbar ist.

Fig. 14 zeigt eine Schnittansicht im Bereich eines Kreises K in Fig. 10.

In der oberen Hälfte von Fig. 14 ist der Schieber 6 in seiner vorgeschobenen Lage dargestellt. Der Schieber 6 weist bei diesem Ausführungsbeispiel in Bezug zu der Kanüle 3 diametral gegenüberliegende Verriegelungszungen auf, wobei in der oberen Hälfte von Fig. 14 eine Verriegelungszunge mit dem Bezugszeichen 26 und in der unteren Hälfte von Fig. 14 eine weitere Verriegelungszunge mit dem Bezugszeichen 26' versehen ist. Die Verriegelungszungen 26, 26' greifen mit radial nach innen gerichteten Vorsprüngen 28, 28' in radiale Ausnehmungen 29, 29' im Inneren des Gehäuses 7 federnd ein. An ihrem dem Entriegelungselement 22 zugewandten Ende weisen die Verriegelungszungen 26, 26' jeweils eine Abschrägung 30, 30' auf. Zwischen dem inneren axialen Ende des Gehäuses 7 und dem Schieber 6 ist eine Wendelfeder 31 angeordnet, die in der vorgeschobenen verriegelten Lage des Schiebers 6 den Schieber 6 entgegen der Einstichrichtung, also in Richtung auf seine zurückgezogene Lage, vorspannt.

Die Entriegelungszungen 24, 25 des Entriegelungselementes 22 erstrecken sich durch radial außen von der vorderen Öffnung 8 des Gehäuses 7 angeordnete Öffnungen 32, 32' und weisen an ihrem der Entriegelungszunge 26, 26' zugewandten Ende jeweils eine Abschrägung 33, 33' auf, mit der die Entriegelungszungen 26, 26' an der jeweiligen Abschrägung 29, 29' der Verriegelungszungen 26, 26' anliegen.

Die Funktionsweise der Kanülenanordnung 1 gemäß den Fig. 10 bis 14 ist wie folgt:

Zum Einstechen der Kanüle 3 befindet sich der Schieber 6 in seiner vorgeschobenen Lage, in der die Verriegelungszungen 26, 26' mit ihren radialen Vorsprüngen 28, 28' in die radialen Ausnehmungen 29, 29' in dem Gehäuse 7 eingreifen und so den in Richtung auf die zurückgezogene Lage vorgespannten Schieber 6 an dem Gehäuse 7 verriegeln. Beispielsweise kann der Schieber 6 bereits beim Zusammenbau der Kanülenanordnung 1 in die verriegelte, vorgeschobene Lage gebracht werden. Es kann jedoch auch entsprechend den Figuren 1 bis 9 ein Betätigungselement vorgesehen sein, mittels dessen der Schieber 6 von Hand verschiebbar ist.

Gelangt beim Einstechen der Kanüle 3 das in Einstichrichtung vordere Ende des Gehäuses 7 in den Bereich der Haut des Patienten, so gelangt das ringförmige Ende 23 des Entriegelungselementes 22 an der Haut zur Anlage und wird bei einem weiteren Einstechen der Kanüle 3 axial in Richtung auf das Gehäuse 7 verschoben, so daß die Entriegelungszungen 24, 25 mit ihren Abschrägungen 30, 30' die Abschrägungen 33, 33' der Verriegelungszungen 26, 27 untergreifen, anheben und auf diese Weise aus den radialen Ausnehmungen 29, 29' in dem Gehäuse 7 herausbewegen, so daß der Schieber 6 entriegelt ist und unter der Federwirkung der Wendelfeder 31 in Richtung eines Pfeiles 34 in seine zurückgezogene Lage bewegt wird.

Auf diese Weise ist eine automatische Entriegelung der Verriegelungsmittel erzielt, so daß ein unerwünscht tiefes Einstechen der Kanüle 3 vermieden und die Handhabung der Kanülenanordnung 1 erleichtert ist.

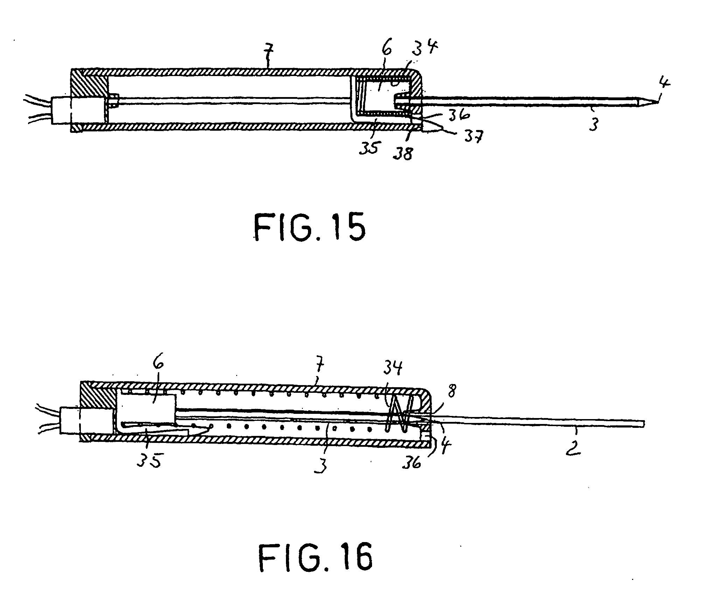

Fig. 15 zeigt ein drittes Ausführungsbeispiel der Erfindung. Es unterscheidet sich von dem in den Fig. 1 bis 9 dargestellten Ausführungsbeispiel dadurch, daß der Schieber 6 durch eine Feder 34 entgegen der Einstechrichtung, also in dem Gehäuse 7 nach hinten, vorgespannt ist. An dem Schieber 6 befindet sich ein Arm 35, dessen Ende durch eine Öffnung 36 in den vorderen Teil des Gehäuses 7 ragt und einen Haken 37 aufweist, der eine vordere Kante 38 des Gehäuses 7 hintergreift und so den Schieber 6 entgegen der Spannung der Feder 34 in der in Fig. 10 dargestellten Lage hält. Der Arm 35 ist elastisch ausgebildet, so daß durch Druck der Haken 37 von der vorderen Kante 38 außer Eingriff gebracht werden kann, so daß der Schieber 6 von der Feder 34 nach hinten gedrückt wird, wobei er die fest mit ihm verbundene Kanüle 3 mitnimmt, so daß beides in eine Lage kommt, die in Fig. 16 dargestellt ist.

Aus Fig. 16 ist ersichtlich, daß die Länge der Kanüle 3 so bemessen ist, daß das vordere spitze Ende 4 der Kanüle 3 innerhalb der vorderen Öffnung 8 des Gehäuses 7 liegt, wodurch der Katheter 2 gegen Beschädigungen durch das vordere spitze Ende 4 der Kanüle 3 geschützt ist, da durch die Halterung der Kanüle 3 in dem Gehäuse 7 eine Relativbewegung von Kanüle 3 und Katheter 2 vermieden ist.

Die Verwendung der Kanülenanordnung gemäß den Figuren 15 und 16 erfolgt in grundsätzlich gleicher Weise wie bei der Kanülenanordnung gemäß den Figuren 1 bis 9 mit dem Unterschied, daß ein Zurückschieben des Schiebers 6 von Hand durch die Kraft der Feder 34 ersetzt ist. Das Entriegeln der durch den Haken 37 und die vordere Kante 38 gebildeten Rastung erfolgt dadurch, daß der vorstehende Haken 37 nach ausreichendem Einstechen der Kanüle 3 gegen die Oberfläche der Haut gedrückt wird. Eine gesonderte Betätigung des Hakens 37 ist dadurch überflüssig.

## Patentansprüche

1. Kanülenanordnung,
- mit einem rohrförmigen Gehäuse (7) mit einem vorderen und hinteren Ende,
- mit einem im Inneren des Gehäuses (7) zwischen einer vorderen und einer hinteren Endlage beweglichen Schieber (6),
- mit einer Kanüle (3), die mit ihrem hinteren Ende fest mit dem Schieber (6) verbunden ist und deren vorderes, eine Spitze zum Einstechen in biologisches Gewebe oder dergleichen aufweisendes Ende sich bis in das vordere Ende des Gehäuses(7) erstreckt, wenn sich der Schieber (6) in der hinteren Endlage befindet,
- mit einem mit dem Gehäuse (7) verbundenen Katheter (2), und
- mit Mitteln zum Zurückbewegen der Kanüle (3),
**dadurch gekennzeichnet,**
**daß** der Katheter (2) entgegen der Einstechrichtung unbeweglich mit dem hinteren Ende des Gehäuses (7) und/oder dem Schieber (6) verbunden ist und sich wenigstens teilweise durch den Schieber (6) und in die Kanüle (3) erstreckt.

2. Kanülenanordnung nach Anspruch 1, **dadurch gekennzeichnet, daß** der Katheter fest mit dem hinteren Ende des Gehäuses verbunden ist und sich bis zum vorderen Ende der Kanüle erstreckt, wenn sich der Schieber (6) in der vorderen Endlage befindet und die Kanüle (3) aus dem vorderen Ende des Gehäuses (7) herausragt.

3. Kanülenanordnung nach Anspruch 1, **gekennzeichnet durch** Verriegelungsmittel zum Verriegeln des Schiebers (6) an dem Gehäuse (7) wenigstens in der vorgeschobenen Lage des Schiebers (6).

4. Kanülenanordnung nach Anspruch 1, **dadurch gekennzeichnet, daß** Mittel zum Zurückziehen der Kanüle (3) von Hand vorgesehen sind.

5. Kanülenanordnung nach Anspruch 4, **dadurch gekennzeichnet, daß** das Gehäuse (7) einen sich in Verschieberichtung der Kanüle (3) erstreckenden Schlitz (17) aufweist, durch den sich ein radialer Vorsprung (10) des Schiebers (6) erstreckt.

6. Kanülenanordnung nach Anspruch 4, **dadurch gekennzeichnet, daß** der Schieber (6) einen um eine Achse quer zu seiner Schieberichtung kippbaren, zweiarmigen Hebel (11) aufweist, dessen vorderer Hebelarm (12) in der vorderen Endlage des Schiebers (6) in einer Raststellung eine Kante (38) am vorderen Ende des Gehäuses (7) hintergreift und der durch Druck auf den hinteren Hebelarm (14) ausrastbar ist.

7. Kanülenanordnung nach Anspruch 6, **dadurch gekennzeichnet, daß** der Schieber (6) und der zweiarmige Hebel (11) über einen Vorsprung (10) miteinander verbunden sind, der durch einen in Längsrichtung des Gehäuses (7) verlaufenden Schlitz (17) in dem Gehäuse (7) ragt.

8. Kanülenanordnung nach Anspruch 1, **dadurch gekennzeichnet, daß** eine Feder (34) vorgesehen ist, die den Schieber (6) in Richtung auf seine hintere Endlage drückt und daß der Schieber (6) in seiner vorderen Endlage durch eine von außerhalb des Gehäuses (7) lösbare Rastung gehalten ist.

9. Kanülenanordnung nach Anspruch 8, **dadurch gekennzeichnet, daß** die Rastung einen Haken (25) aufweist, der mit dem Schieber (6) verbunden ist, in der vorderen Endlage des Schiebers (6) eine Kante (38) am vorderen Ende des Gehäuses (7) hintergreift und in Entriegelungsrichtung nachgiebig ist.

10. Kanülenanordnung nach Anspruch 9, **dadurch gekennzeichnet, daß** der Haken über das vordere Ende des Gehäuses (7) vorsteht, derart, daß er bei Einstechen der Kanüle (3) gegen die Gewebeoberfläche stößt und den Haken (25) entriegelt.

11. Kanülenanordnung nach Anspruch 3, **dadurch gekennzeichnet, daß** die Verriegelungsmittel eine mit dem Ansatz verbundene Verriegelungszunge (26) aufweisen, die in Verriegelungslage mit einem radialen Vorsprung (28) federnd in eine radiale Ausnehmung (29) im Inneren des Gehäuses (7) eingreift und daß die Entriegelungsmittel ein relativ zu dem Gehäuse (7) axial verschiebliches Entriegelungselement (22) aufweisen, dessen vorderes Ende axial über das vordere Ende des Gehäuses (7) vorsteht und dessen hinteres Ende eine Entriegelungszunge (24) aufweist, die sich in das Innere des Gehäuses (7) erstreckt und bei axialer Verschiebung des Entriegelungselementes (22) in Richtung auf das Gehäuse (7) die Verriegelungszunge (26) untergreift und aus der Ausnehmung (29) herausbewegt.

12. Kanülenanordnung nach Anspruch 11, **dadurch gekennzeichnet, daß** die Entriegelungszunge (24) und die Verriegelungszunge (26) in Ruhelage mit Abschrägungen (31, 33) aneinander anliegen, derart, daß die Entriegelungszunge (24) bei axialer Verschiebung des Entriegelungselementes (22) in Richtung auf das Gehäuse (7) die Verriegelungszunge (26) anhebt.

13. Kanülenanordnung nach Anspruch 11, **dadurch gekennzeichnet, daß** jeweils wenigstens zwei Entriegelungszungen (24, 25) und zugeordnete Verriegelungszungen (26, 27) an in bezug zu der Kanüle (3) diametral gegenüberliegenden Stellen des Entriegelungselementes (22) bzw. des Schiebers (6) vorgesehen sind.

14. Kanülenanordnung nach Anspruch 11, **dadurch gekennzeichnet, daß** das vordere Ende (23) des Entriegelungselementes (22) im wesentlichen ringförmig ausgebildet ist.

15. Kanülenanordnung nach Anspruch 8, **dadurch gekennzeichnet, daß** die Feder eine zwischen dem Gehäuseteil (7) und dem Schieber (6) angeordnete Wendelfeder (31), insbesondere eine zu der Kanüle (3) koaxiale Druckfeder, ist.

16. Kanülenanordnung nach Anspruch 1, **gekennzeichnet durch** Haltemittel zum Halten des Gehäuses (7) auf einer Körperoberfläche, beispielsweise der Haut (19) eines Menschen.

17. Kanülenanordnung nach Anspruch 16, **dadurch gekennzeichnet, daß** die Haltemittel auf der Außenseite des Gehäuses (7) angeordnete Adhäsivmittel, insbesondere einen Klebestreifen, aufweisen.

18. Kanülenanordnung nach Anspruch 1, **dadurch gekennzeichnet, daß** das Gehäuse (7) eine Abflachung (18) zur Anlage an der Körperoberfläche, beispielsweise der Haut eines Menschen, aufweist.

19. Kanülenanordnung nach Anspruch 1, **dadurch gekennzeichnet, daß** die vordere Öffnung (8) des Gehäuses (7) eine lichte Weite aufweist, die im wesentlichen dem Außendurchmesser der Kanüle (3) entspricht.

20. Kanülenanordnung nach Anspruch 1, **dadurch gekennzeichnet, daß** der Schieber (6) eine Hemmung für den Katheter (2) aufweist, die in Einstechrichtung wirkt und den Katheter beim Vorschieben des Schiebers (6) mitnimmt.

## Claims

1. A cannula system,
- having a tubular housing (7) with a front and a rear end,
- having a slider (6) displaceable inside the housing (7) between a front and a rear limit position,
- having a cannula (3), which is securely connected by its rear end to the slider (6) and the front end of which, comprising a tip for piercing into biological tissue or the like, extends right into the front end of the housing (7) when the slider (6) is in the rear limit position,
- having a catheter (2) connected to the housing (7), and
- having means for moving back the cannula (3),
**characterised in that**, in the direction opposite the direction of piercing, the catheter (2) is immovably connected to the rear end of the housing (7) and/or the slider (6) and extends at least partly through the slider (6) and into the cannula (3).

2. A cannula system according to Claim 1,
**characterised in that** the catheter is securely connected to the rear end of the housing and extends to the front end of the cannula when the slider (6) is in the front limit position and the cannula (3) projects from the front end of the housing (7).

3. A cannula system according to Claim 1,
**characterised by** locking means for locking the slider (6) on the housing (7) at least in the advanced position of the slider (6).

4. A cannula system according to Claim 1,
**characterised in that** means are provided for manually retracting the cannula (3).

5. A cannula system according to Claim 4,
**characterised in that** the housing (7) comprises a slot (17) which extends in the direction of displacement of the cannula (3) and through which extends a radial projection (10) of the slider (6).

6. A cannula system according to Claim 4,
**characterised in that** the slider (6) comprises a two-armed lever (11) tiltable around an axis transversely to its direction of displacement, the front lever arm (12) of which engages behind an edge (38) at the front end of the housing (7) in the front limit position of the slider (6) in a detent position, and which can be disengaged by pressing on the rear lever arm (14).

7. A cannula system according to Claim 6,
**characterised in that** the slider (6) and the two-armed lever (11) are connected to one another by a protrusion (10), which projects through a slit (17) in the housing (7) running in the longitudinal direction of the housing (7).

8. A cannula system according to Claim 1,
**characterised in that** a spring (34) is provided, which presses the slider (6) towards its rear limit position and **in that** the slider (6) is retained in its front limit position by a detent means which can be released from outside the housing (7).

9. A cannula system according to Claim 8,
**characterised in that** the detent means comprises a hook (25), which is connected to the slider (6), in the front limit position of the slider (6) engages behind an edge (38) on the front end of the housing (7) and is resilient in the direction of disengagement.

10. A cannula system according to Claim 9,
**characterised in that** the hook projects beyond the front end of the housing (7) in such a manner that it abuts against the tissue surface and disengages the hook (25) when the cannula pierces.

11. A cannula system according to Claim 3,
**characterised in that** the locking means comprise a locking blade (26) which is connected to the catch and which in the locking position engages resiliently by a radial protrusion (28) into a radial recess (29) in the interior of the housing (7),
and **in that** the unlocking means comprise an unlocking element (22) which is axially displaceable in relation to the housing (7) and the front end of which projects axially beyond the front end of the housing (7) and the rear end of which comprises an unlocking blade (24), which extends into the interior of the housing (7) and upon the axial displacement of the unlocking element (22) towards the housing (7) engages under the locking blade (26) and moves it out of the recess (29).

12. A cannula system according to Claim 11,
**characterised in that** the unlocking blade (24) and the locking blade (26) rest against each other in the rest position by means of bevels (31, 33) in such a manner that the unlocking blade (24) raises the locking blade (26) upon the axial displacement of the locking element (2) towards the housing (7).

13. A cannula system according to Claim 11,
**characterised in that** in each case at least two unlocking blades (24, 25) and associated locking blades (26, 27) are provided at sites of the unlocking element (22) or of the slider (6) that are diametrically opposite in relation to the cannula (3).

14. A cannula system according to Claim 11,
**characterised in that** the front end (23) of the unlocking element (22) has a substantially annular design.

15. A cannula system according to Claim 8,
**characterised in that** the spring is a helical spring (32) disposed between the housing part (7) and the slider (6), in particular a compression spring coaxial with the cannula (3).

16. A cannula system according to Claim 1,
**characterised by** means for retaining the housing (7) on a body surface, for example the skin (19) of a human being.

17. A cannula system according to Claim 16,
**characterised in that** the retaining means comprise adhesive means disposed on the outside of the housing (7), in particular an adhesive strip.

18. A cannula system according to Claim 1,
**characterised in that** the housing (7) comprises a flattened portion (18) to rest on the body surface, for example the skin of a human being.

19. A cannula system according to Claim 1,
**characterised in that** the front opening (8) of the housing (7) comprises an inside width that substantially corresponds to the external diameter of the cannula (3).

20. A cannula system according to Claim 1,
**characterised in that** the slider (6) comprises a catch for the catheter (2), which acts in the piercing direction and entrains the catheter when the slider (6) is being advanced.

## Revendications

1. Système de canule
- comportant un boîtier (7) de forme tubulaire avec une extrémité avant et une extrémité arrière,
- comportant un coulisseau (6) déplaçable à l'intérieur du boîtier (7), entre une position de fin de course avant et une position de fin de course arrière,
- comportant une canule (3) qui, par son extrémité arrière, est solidaire du coulisseau (6) et dont l'extrémité avant, présentant une pointe destinée à être piquée dans un tissu biologique ou similaire, s'étend jusque dans l'extrémité avant du boîtier (7) lorsque le coulisseau (6) se trouve dans sa position de fin de course arrière,
- comportant un cathéter (2) relié au boîtier (7), et
- comportant des moyens pour replacer la canule (3),
**caractérisé**
**en ce que** le cathéter (2) est relié, de manière non mobile dans le sens contraire au sens d'introduction, à l'extrémité arrière du boîtier (7) et/ou du coulisseau (6) et s'étend au moins en partie à travers le coulisseau (6) et dans la canule (3).

2. Système de canule selon la revendication 1, **caractérisé en ce que** le cathéter est solidaire de l'extrémité arrière du boîtier et s'étend jusqu'à l'extrémité avant de la canule lorsque le coulisseau (6) se trouve dans sa position de fin de course avant et la canule (3) ressort de l'extrémité avant du boîtier (7).

3. Système de canule selon la revendication 1, **caractérisé par** des moyens de verrouillage pour verrouiller le coulisseau (6) sur le boîtier (7), au moins dans la position avancée du coulisseau (6).

4. Système de canule selon la revendication 1, **caractérisé en ce que** des moyens sont prévus pour retirer la canule (3) à la main.

5. Système de canule selon la revendication 4, **caractérisé en ce que** le boîtier (7) présente une fente (17) s'étendant dans la direction de déplacement de la canule (3) à travers laquelle s'étend une saillie radiale (10) du coulisseau (6).

6. Système de canule selon la revendication 4, **caractérisé en ce que** le coulisseau (6) comporte un levier (11) à deux bras pouvant basculer autour d'un axe, transversalement à sa direction de coulissement, dont le bras avant (12), en position de fin de course avant du coulisseau (6), dans une position d'encliquetage, passe derrière un bord (38) de l'extrémité avant du boîtier (7), et qui peut être dégagé par pression sur le bras de levier arrière (14).

7. Système de canule selon la revendication 6, **caractérisé en ce que** le coulisseau (6) et le levier (11) à deux bras sont reliés entre eux par une saillie (10) qui s'étend dans le boîtier (7), à travers une fente (17) s'étendant dans la direction longitudinale du boîtier (7).

8. Système de canule selon la revendication 1, **caractérisé en ce qu'**il est prévu un ressort (34) qui presse le coulisseau (6) en direction de sa position de fin de course arrière, et **en ce que** le coulisseau (6), dans sa position de fin de course avant, est maintenu par un organe d'encliquetage qui peut être déverrouillé de l'extérieur du boîtier (7).

9. Système de canule selon la revendication 8, **caractérisé en ce que** l'organe d'encliquetage comporte un crochet (25) qui est relié au coulisseau (6), qui, en position de fin de course avant du coulisseau (6), coopère avec une butée (38) au niveau de l'extrémité avant du boîtier (7), et qui est flexible dans le sens du déverrouillage.

10. Système de canule selon la revendication 9, **caractérisé en ce que** le crochet dépasse de l'extrémité avant du boîtier (7) de manière que lorsque l'on enfonce la canule (3), il bute contre la surface du tissu et déverrouille le crochet (25).

11. Système de canule selon la revendication 3, **caractérisé en ce que** les moyens de verrouillage comportent une languette de verrouillage (26) reliée à l'appendice, laquelle, en position de verrouillage, s'engage de manière élastique, par une saillie radiale (28), dans un évidement radial (29) à l'intérieur du boîtier (7), et **en ce que** les moyens de déverrouillage comportent un élément de déverrouillage (22) pouvant coulisser axialement par rapport au boîtier (7), dont l'extrémité avant dépasse axialement de l'extrémité avant du boîtier (7), et dont l'extrémité arrière comporte une languette de déverrouillage (24) qui s'étend à l'intérieur du boîtier (7) et qui, lors du coulissement axial de l'élément de déverrouillage (22), en direction du boîtier (7), passe sous la languette de verrouillage (26) et la déplace à l'extérieur de l'évidement (29).

12. Système de canule selon la revendication 11, **caractérisé en ce que** la languette de déverrouillage (24) et la languette de verrouillage (26) s'appliquent en position de repos l'une contre l'autre par des chanfreins (31, 33), de manière que la languette de déverrouillage (24) soulève la languette de verrouillage (26) lors du coulissement axial de l'élément de déverrouillage (22) en direction du boîtier (7).

13. Système de canule selon la revendication 11, **caractérisé en ce qu'**au moins deux languettes de déverrouillage (24, 25) et languettes de verrouillage (26, 27) associées sont prévues en des points, diamétralement opposés l'un à l'autre par rapport à la canule (3), de l'élément de déverrouillage (22) ou du coulisseau (6).

14. Système de canule selon la revendication 11, **caractérisé en ce que** l'extrémité avant (23) de l'élément de déverrouillage (22) est réalisée sensiblement en forme d'anneau.

15. Système de canule selon la revendication 8, **caractérisé en ce que** le ressort est un ressort spiral (31), disposé entre la partie de boîtier (7) et le coulisseau (6), en particulier un ressort de pression coaxial à la canule (3).

16. Système de canule selon la revendication 1, **caractérisé par** des moyens de retenue pour retenir le boîtier (7) sur une surface corporelle, par exemple la peau (19) d'une personne.

17. Système de canule selon la revendication 16, **caractérisé en ce que** les moyens de retenue présentent des moyens adhésifs, en particulier une bande adhésive, disposés sur le côté extérieur du boîtier (7).

18. Système de canule selon la revendication 1, **caractérisé en ce que** le boîtier (7) présente un méplat (18) destiné à s'appliquer contre la surface corporelle, par exemple la peau d'une personne.

19. Système de canule selon la revendication 1, **caractérisé en ce que** l'ouverture avant (8) du boîtier (7) présente une ouverture libre qui correspond sensiblement au diamètre extérieur de la canule (3).

20. Système de canule selon la revendication 1, **caractérisé en ce que** le coulisseau (6) comporte un organe d'arrêt pour le cathéter (2), qui agit dans le sens d'enfoncement et entraîne le cathéter lors de l'avance du coulisseau (6).
